# EUROPÄISCHE PATENTSCHRIFT

(11) **EP 1 682 542 B1**
(45) Veröffentlichungstag und Bekanntmachung des Hinweises auf die Patenterteilung: **30.12.2009**
(21) Anmeldenummer: 04791029.4
(22) Anmeldetag: 29.10.2004
(51) Int. Cl.: C07D 451/10, A61K 31/46, A61P 11/00

(54) **NEUES KRISTALLINES ANHYDRAT MIT ANTICHOLINERGER WIRKSAMKEIT**
NOVEL CRYSTALLINE ANHYDRIDE WITH ANTICHOLINERGIC EFFECT
NOUVEL ANHYDRATE CRISTALLIN A EFFET ANTICHOLINERGIQUE

(30) Priorität: 03.11.2003 EP 03025077
(43) Veröffentlichungstag der Anmeldung: 26.07.2006
(73) Patentinhaber: Boehringer Ingelheim International GmbH, 55216 Ingelheim am Rhein (DE); Boehringer Ingelheim Pharma GmbH & Co.KG, 55216 Ingelheim am Rhein (DE)
(72) Erfinder: PFRENGLE, Waldemar, 88400 Biberach (DE); SIEGER, Peter, 88441 Mittelbiberach (DE)
(74) Vertreter: Hammann, Heinz
(86) Internationale Anmeldenummer: PCT/EP2004/012269
(87) Internationale Veröffentlichungsnummer: WO 2005/042527

(56) Entgegenhaltungen:
- EP-A- 0 418 716
- WO-A-02/30928
- STAHL, P.H.; WERMUTH, C.G.: "Handbook of Pharmaceutical Salts Properties, Selection and Use" 2002, WILEY-VCH , WEINHEIM-NEW YORK , XP002268934 Seite 98
- DISSE, B. ET AL.: "Tiotropium (Spiriva TM) Mechanistical Considerations and Clinical Profile in Obstructive Lung Disease" LIFE SCIENCES, Bd. 64, Nr. 6, 1999, XP002268933

## Beschreibung

Die Erfindung betrifft ein neues, kristallines Anhydrat des Tiotropiumbromids, Verfahren zu dessen Herstellung sowie dessen Verwendung zur Herstellung eines Arzneimittels zur Behandlung von Atemwegserkrankungen, insbesondere zur Behandlung von COPD (chronic obstructive pulmonary disease = chronisch obstruktive Lungenerkrankung) und Asthma.

### Hintergrund der Erfindung

Tiotropiumbromid ist aus der Europäischen Patentanmeldung EP 4181 716 A1 bekannt und weist die folgende chemische Struktur auf:

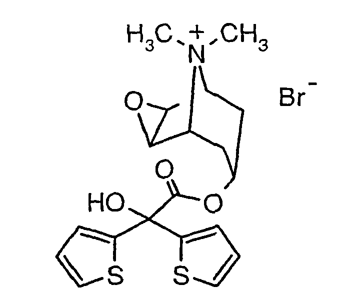

Tiotropiumbromid stellt ein hoch wirksames Anticholinergikum mit langanhaltender Wirkdauer dar, welches zur Therapie von Atemwegserkrankungen, insbesondere von COPD (chronic obstructive pulmonary disease = chronisch obstruktive Lungenerkrankung) und Asthma Verwendung finden kann. Unter Tiotropium ist das freie Ammoniumkation zu verstehen.

Die Applikation von Tiotropiumbromid erfolgt vorzugsweise auf inhalativem Wege. Hierbei können geeignete Inhaltionspulver, die in geeignete Kapseln (Inhaletten) abgefüllt mittels entsprechender Pulverinhalatoren appliziert werden, zum Einsatz kommen. Alternativ dazu kann eine inhalative Anwendung auch durch Applikation geeigneter Inhalationsaerosole erfolgen. Hierzu zählen auch pulverförmige Inhalationsaerosole, die beispielsweise HFA134a, HFA227 oder deren Gemisch als Treibgas enthalten.

Die richtige Herstellung der vorgenannten, zur inhalativen Verabreichung eines Arzneiwirkstoffes verwendbaren Zusammensetzungen stützt sich auf verschiedene Parameter, die mit der Beschaffenheit des Arzneiwirkstoffes selbst verbunden sind. Bei Arzneimitteln, die wie Tiotropiumbromid in Form von Inhalationspulvern oder Inhalationsaerosolen zur Anwendung gelangen, wird der kristalline Wirkstoff in gemahlener (mikronisierter) Form zur Herstellung der Formulierung verwendet. Da für die pharmazeutische Qualität einer Arzneimittelformulierung stets dieselbe kristalline Modifikation des Wirkstoffs gewährleistet sein muß, sind vor diesem Hintergrund an die Stabilität und Eigenschaften des kristallinen Wirkstoffes erhöhte Anforderungen zu stellen. Es ist besonders wünschenswert, den Wirkstoff in Form einer einheitlichen und klar definierten Kristallmodifikation bereitzustellen. Es ist ferner besonders wünschenswert, den Wirkstoff in einer kristallinen Form bereitzustellen, die auch bei längerer Lagerung durch ein hohes Maß an Stabilität gekennzeichnet ist. Je geringer eine Kristallmodifikation beispielsweise zur Aufnahme von Feuchtigkeit neigt, desto größer ist die physikalische Stabilität ihrer Kristallstruktur.

Die Aufgabe der Erfindung besteht somit in der Bereitstellung einer neuen, stabilen Kristallform der Verbindung Tiotropiumbromid, die den vorstehend genannten hohen Anforderungen, die an einen Arzneimittelwirkstoff zu richten sind, genügen. Es ist insbesondere Aufgabe der vorliegenden Erfindung, eine Kristallmodifikation des Tiotropiumbromids bereitzustellen, die nur ein geringes hygroskopisches Verhalten aufweist.

### Detaillierte Beschreibung der Erfindung

Es wurde gefunden, daß Tiotropiumbromid je nach Wahl der Bedingungen, die bei der Reinigung des nach der technischen Herstellung erhaltenen Rohprodukts angewendet werden können, in unterschiedlichen kristallinen Modifikationen anfällt.

Es wurde gefunden, daß diese unterschiedlichen Modifikationen maßgeblich durch Wahl der zur Kristallisation eingesetzten Lösemittel sowie durch Wahl der beim Kristallisationsprozess gewählten Verfahrensbedingungen gezielt erhalten werden können.

Es wurde überraschenderweise festgestellt, daß ausgehend vom Monohydrat des Tiotropiumbromids, welches durch die Wahl spezifischer Reaktionsbedingungen in kristalliner Form erhalten werden kann und in der WO 02/30928 erstmals im Stand der Technik beschrieben wurde, eine wasserfreie Kristallmodifikation des Tiotropiumbromids erhalten werden kann, die den eingangs genannten hohen Anforderungen genügt und somit die der vorliegenden Erfindung zugrunde liegende Aufgabe löst. Dementsprechend betrifft die vorliegende Erfindung dieses kristalline, wasserfreie Tiotropiumbromid. Eine im Rahmen der vorliegenden Erfindung gegebenenfalls erfolgende Bezugnahme auf die Bezeichnung Tiotropiumbromid-anhydrat ist als Bezugnahme auf das erfindungsgemäße kristalline, wasserfreie Tiotropiumbromid anzusehen.

Ein weiterer Aspekt der vorliegenden Erfindung betrifft ein Herstellverfahren der neuen kristallinen Form des wasserfreien Tiotropiumbromids, welches in beispielhafter Form im nachstehenden experimentellen Teil erläutert wird.

Das erfindungsgemäße wasserfreie Tiotropiumbromid zeichnet sich insbesondere durch ein geringes hygroskopisches Verhalten aus, welches ein hohes Maß an Stabilität der Kristallmodifikation gewährleistet. Das erfindungsgemäße kristalline Tiotropiumbromid-anhydrat ist hoch kristallin und eignet sich deshalb besonders gut zur Darstellung von inhalativ zu applizierenden Arzneimittelformulierungen.

Die vorliegende Erfindung betrifft ferner die Verwendung des erfindungsgemäßen kristallinen Tiotropiumbromid-anhydrats zur Herstellung eines Arzneimittels zur Behandlung von Atemwegserkrankungen, insbesondere zur Behandlung von COPD und/oder Asthma.

Die folgenden Beispiele dienen einer weitergehenden Erläuterung der vorliegenden Erfindung, ohne den Umfang der Erfindung allerdings auf die nachfolgenden beispielhaften Ausführungsformen zu beschränken.

### A. I. Ausgangsmaterialien

### Tiotropiumbromidmonohydrat:

Tiotropiumbromid, welches beispielsweise gemäß der in der europäischen Patentanmeldung EP 418 716 beschriebenen Vorgehensweise erhalten wurde, wurde entsprechend der Offenbarung der WO 02/30928 in kristallines TiotropiumbromidMonohydrat überführt. Dieses dient als Ausgangsverbindung zur Darstellung des erfindungsgemäßen Tiotropiumbromid-anhydrats.

### A. II. erfinduneseemäße Synthesebeispiele

### Beispiel 1:

10,0g Tiotropiumbromidmonohydrat wurden in 100 ml Wasser aufgenommen und bei Siedetemperatur gelöst. Anschließend erfolgte die Zugabe von 80,0g Ammoniumfluorid. Nach 18 Stunden Rühren bei etwa 20-25°C wird das auskristallisierte Produkt isoliert und bei 70°C getrocknet. Das erhaltene Rohprodukt wird in 25 ml Methanol bei Siedetemperatur gelöst, heiß filtriert und auf Raumtemperatur (ca. 20-25°C) abgekühlt. Das nach Ankratzen der Kolbenwand (mittels Glasstab) sofort kristallierende Produkt wird isoliert und bei 50°C getrocknet.
Ausbeute: 7,35 g Tiotropiumbromid-anhydrat, weißer Feststoff.

### Beispiel 2:

4,39g Tiotropiumbromidmonohydrat wurden in 25 ml Methanol aufgenommen und bei Siedetemperatur gelöst. Die erhaltene klare Lösung wird mit wenigen Kristallen des nach Beispiel 1 erhaltenen kristallinen Tiotropiumbromid-anhydrats angeimpft. Dabei kristallisiert das Produkt bereits in der Wärme aus. Nach langsamem Abkühlen auf Raumtemperatur (ca. 20-25°C) wird der erhaltene Niederschlag abfiltriert und bei 50°C getrocknet. Ausbeute: 3,47 g Tiotropiumbromid-anhydrat, weißer Feststoff.

### A. III. Charakterisierung des erfindungsgemäßen Tiotropiunibromidanhydrats

Das gemäß vorstehender Methodik erhaltene Tiotropiumbromid-anhydrat ist hochkristallin. Es wurde mit Hilfe der Röntgenpulverbeugung weitergehend untersucht. Zur Aufnahme des nachstehend aufgeführten Röntgenpulverdiagramms wurde wie folgt verfahren.

Das Röntgenpulverdiagramm wurde im Rahmen der vorliegenden Erfindung aufgenommen mittels eines Bruker D8 Advanced mit einem OED (= ortsempfindlicher Detektor) (CuK_{α} - Strahlung, λ =1.5418 Å, 30 kV, 40 mA).

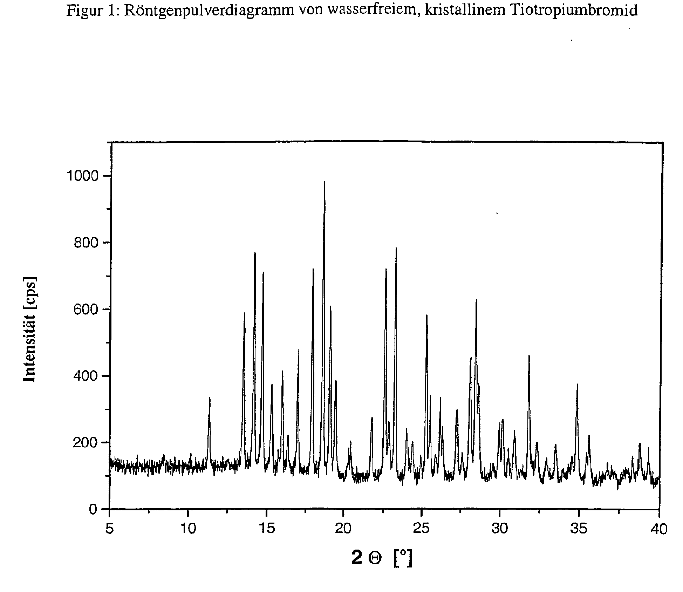

Das für das erfindungsgemäße Tiotropiumbromid-anhydrat erhaltene Röntgenpulverdiagramm ist in Figur 1 dargestellt.

In nachstehender Tabelle 1 sind die charakteristischen Peaks und normierten Intensitäten aufgelistet.

**Tabelle 1:**

| **2 Θ [°]** | **dₕₖₗ [Å]** | **Intensität [%]** |
|---|---|---|
| 8,39 | 10,53 | 4 |
| 11,33 | 7,80 | 27 |
| 13,50 | 6,55 | 53 |
| 14,13 | 6,26 | 65 |
| 14,70 | 6,02 | 73 |
| 15,28 | 5,79 | 31 |
| 15,72 | 5,63 | 7 |
| 15,98 | 5,54 | 33 |
| 16,32 | 5,43 | 11 |
| 16,95 | 5,23 | 45 |
| 17,90 | 4,95 | 76 |
| 18,55 | 4,78 | 100 |
| 19,07 | 4,65 | 62 |
| 19,46 | 4,56 | 36 |
| 20,32 | 4,37 | 10 |
| 20,40 | 4,35 | 12 |
| 21,75 | 4,08 | 23 |
| 22,60 | 3,93 | 77 |
| 22,83 | 3,89 | 20 |
| 23,21 | 3,83 | 86 |
| 24,01 | 3,70 | 17 |
| 24,40 | 3,64 | 13 |
| 24,93 | 3,57 | 8 |
| 25,27 | 3,52 | 60 |
| 25,55 | 3,48 | 28 |
| 25,95 | 3,43 | 7 |
| 26,21 | 3,40 | 30 |
| 26,38 | 3,38 | 18 |
| 27,26 | 3,27 | 24 |
| 27,60 | 3,23 | 8 |
| 28,07 | 3,18 | 44 |
| 28,38 | 3,14 | 67 |
| 28,58 | 3,12 | 35 |
| 29,93 | 2,98 | 19 |
| 30,18 | 2,96 | 21 |
| 30,52 | 2,93 | 10 |
| 30,91 | 2,89 | 17 |
| 31,82 | 2,81 | 46 |
| 32,32 | 2,77 | 13 |
| 32,94 | 2,72 | 7 |
| 33,49 | 2,67 | 13 |
| 34,80 | 2,58 | 31 |

In vorstehender Tabelle steht der Wert "2 Θ [°]" für den Beugungswinkel in Grad und der Wert " dₕₖₗ [Å]" für die bestimmten Gitterebenenabstände in Å.

Die vorliegende Erfindung betrifft dementsprechend kristallines Tiotropiumbromid-Anhydrat, welches dadurch gekennzeichnet ist, dass es im Röntgenpulverdiagramm unter anderen die charakteristischen Werte d= 6,02 Å; 4,95 Å; 4,78 Å; ; 3,93 Ä und 3,83 Å aufweist.

### B. Formulierungen enthaltend das erfindungsgemäße Tiotropiumbromid-anhydrat

Das erfindungsgemäße kristalline Tiotropiumbromid-anhydrat ist hoch kristallin und eignet sich deshalb besonders gut zur Darstellung von beispielsweise inhalativ zu applizierenden Arzneimittelformulierungen wie Inhalationspulvern oder beispielsweise auch treibgashaltigen Aerosolformulierungen, insbesonders Inhalationspulvern und treibgashaltigen Aerosolsuspensionsformulierungen.

### B.1. Inhalationspulver

Die vorliegende Erfindung betrifft ferner Inhalationspulver enthaltend 0,001 bis 3 % Tiotropium in Form des erfindungsgemäßen kristallinen Tiotropiumbromid-anhydrats im Gemisch mit einem physiologisch unbedenklichen Hilfsstoff. Unter Tiotropium ist dabei das Ammoniumkation zu verstehen.
Erfindungsgemäß bevorzugt sind Inhalationspulver, die 0,01 bis 2 % Tiotropium enthalten. Besonders bevorzugte Inhalationspulver enthalten Tiotropium in einer Menge von etwa 0,03 bis 1 %, bevorzugt 0,05 bis 0,6 %, besonders bevorzugt 0,06 bis 0,3 %. Erfindungsgemäß von besonderer Bedeutung sind schließlich Inhalationspulver, die etwa 0,08 bis 0,22 % Tiotropium enthalten.
Vorstehend genannte Anteile an Tiotropium sind bezogen auf die Menge enthaltenen Tiotropiumkations.

Die Hilfsstoffe, die im Sinne der vorliegenden Erfindung zur Anwendung gelangen, werden durch geeignetes Mahlen und/oder Sieben nach gängigen, im Stand der Technik bekannten Verfahren hergestellt. Gegebenenfalls handelt es sich bei den erfindungsgemäß zum Einsatz gelangenden Hilfsstoffen auch um Hilfsstoffgemische, die durch Mischen von Hilfsstofffraktionen unterschiedlicher mittlerer Teilchengröße erhalten werden.

Als physiologisch unbedenkliche Hilfsstoffe, die zur Darstellung der im Rahmen der erfindungsgemäßen Inhaletten zur Anwendung gelangenden Inhalationspulver Verwendung finden können, seien beispielsweise genannt Monosaccharide (z.B. Glucose, Fructose, Arabinose), Disaccharide (z.B. Lactose, Saccharose, Maltose, Trehalose), Oligo- und Polysaccharide (z.B. Dextrane, Dextrine, Maltodextrin, Stärke, Cellulose), Polyalkohole (z.B. Sorbit, Mannit, Xylit), Cyclodextrine (z. B. α-Cyclodextrin, β-Cyclodextrin, χ-Cyclodextrin, Methyl-β-Cyclodextrin, Hydroxypropyl-β-Cyclodextlin), Aminosäuren (z.B. Argininhydrochlorid) oder auch Salze (z.B. Natriumchlorid, Calciumcarbonat), oder Mischungen davon Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt als Hilfsstoff Lactose, höchst bevorzugt Lactosemonohydrat zur Anwendung.

Die Hilfsstoffe weisen im Rahmen der erfindungsgemäßen Inhalationspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfsstoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Die Bestimmung der mittleren Teilchengröße kann nach im Stand der Technik bekannten Verfahren erfolgen (siehe beispielsweise WO 02/30389, Abschnitte A und C). Schließlich wird zur Herstellung der erfindungsgemäßen Inhalationspulver mikronisiertes kristallines Tiotropiumbromid-anhydrat, welches vorzugsweise durch eine mittlere Teilchengröße von 0,5 bis 10µm, besonders bevorzugt von 1 bis 5µm gekennzeichnet ist, der Hilfsstoffmischung beigemischt. (siehe beispielsweise WO 02/30389, Abschnitt B).Verfahren zum Mahlen und Mikronisieren von Wirkstoffen sind im Stand der Technik bekannt.

Wird als Hilfsstoff keine spezifisch hergestellte Hilfsstoffmischung verwendet, gelangen besonders bevorzugt solche Hilfsstoffe zur Anwendung, die eine mittlere Teilchengröße von 10 - 50 µm, einen 10 %-Feinanteil von 0,5 bis 6 µm aufweisen.
Dabei wird unter der mittleren Teilchengröße im hier verwendeten Sinne der 50 % - Wert aus der Volumenverteilung gemessen mit einem Laserdiffraktometer nach der Trockendispersionsmethode verstanden. Die Bestimmung der mittleren Teilchengröße kann nach im Stand der echnik bekannten Verfahren erfolgen (siehe beispielsweise WO 02/30389, Abschnitte A und B). Analog ist unter dem 10 % - Feinanteil im hier verwendeten Sinne der 10 % - Wert aus der mit einem Laserdiffraktometer gemessenen Volumenverteilung zu verstehen. Mit anderen Worten steht im Sinne der vorliegenden Erfindung der 10%-Feinanteil-Wert für die Teilchengröße, unterhalb derer 10% der Teilchenmenge liegt (bezogen auf Volumenverteilung).

Bei den im Rahmen der vorliegenden Erfindung genannten prozentualen Angaben, handelt es sich stets um Gewichtsprozent, soweit nichts Gegenteiliges spezifisch hervorgehoben wird.

In besonders bevorzugten Inhalationspulvern ist der Hilfsstoff durch eine mittlere Teilchengröße von 12 bis 35 µm, besonders bevorzugt von 13 bis 30 µm gekennzeichnet. Ferner sind insbesondere solche Inhalationspulver besonders bevorzugt, in denen der 10 %-Feinanteil etwa 1 bis 4 µm, bevorzugt etwa 1,5 bis 3 µm beträgt.

Die erfindungsgemäßen Inhalationspulver sind entsprechend der der vorliegenden Erfindung zugrunde liegenden Aufgabe gekennzeichnet durch ein hohes Maß an Homogenität im Sinne der Einzeldosierungsgenauigkeit. Diese liegt in einem Bereich von < 8 %, bevorzugt < 6 % , besonders bevorzugt < 4 %.

Nach Einwaage der Ausgangsmaterialien erfolgt die Herstellung der Inhalationspulver aus dem Hilfsstoff und dem Wirkstoff unter Verwendung von im Stand der Technik bekannten Verfahren an. Hierbei sei beispielsweise auf die Offenbarung der WO 02/30390 verwiesen. Die erfindungsgemäßen Inhalationspulver sind dementsprechend beispielsweise gemäß der nachfolgend beschriebenen Vorgehensweise erhältlich. Bei den nachstehend beschriebenen Herstellverfahren werden die genannten Komponenten in den Gewichtsanteilen eingesetzt, wie sie in den zuvor beschriebenen Zusammensetzungen der Inhalationspulver beschrieben wurden.

Zunächst werden der Hilfsstoff und der Wirkstoff in einen geeigneten Mischbehälter eingebracht. Der verwendete Wirkstoff weist eine mittlere Teilchengröße von 0,5 bis 10 µm, vorzugsweise von 1 bis 6 µm, besonders bevorzugt von 2 bis 5 µm auf. Die Zugabe des Wirkstoffs und des Hilfsstoffs erfolgt vorzugsweise über ein Sieb oder einen Siebgranulator mit einer Maschenweite von 0,1 bis 2 mm, besonders bevorzugt 0,3 bis 1 mm, höchst bevorzugt 0,3 bis 0,6 mm. Vorzugsweise wird der Hilfsstoff vorgelegt und anschließend der Wirkstoff in den Mischbehälter eingebracht. Bevorzugt erfolgt bei diesem Mischverfahren die Zugabe der beiden Komponenten portionsweise. Besonders bevorzugt ist das abwechselnde, schichtweise Einsieben der beiden Komponenten. Der Mischvorgang des Hilfsstoffs mit dem Wirkstoff kann bereits während der Zugabe der beiden Komponenten erfolgen. Vorzugsweise wird allerdings erst nach dem schichtweisen Einsieben der beiden Bestandteile gemischt.

Die vorliegende Erfindung betrifft ferner die Verwendung der erfindungsgemäßen Inhalationspulver zur Herstellung eines Arzneimittels zur Behandlung von Atemwegserkrankungen, insbesondere zur Behandlung von COPD und/oder Asthma.

Die erfindungsgemäßen Inhalationspulver können beispielsweise mittels Inhalatoren appliziert werden, die eine einzelne Dosis aus einem Vorrat mittels einer Messkammer (z.B. gemäß US 4570630A) oder über andere apparative Vorrichtungen (z.B. gemäß DE 36 25 685 A) dosieren. Vorzugsweise werden die erfindungsgemäßen Inhalationspulver allerdings in Kapseln abgefüllt (zu so genannten Inhaletten), die in Inhalatoren wie beispielsweise in der WO 94/28958 beschrieben, zur Anwendung gelangen.

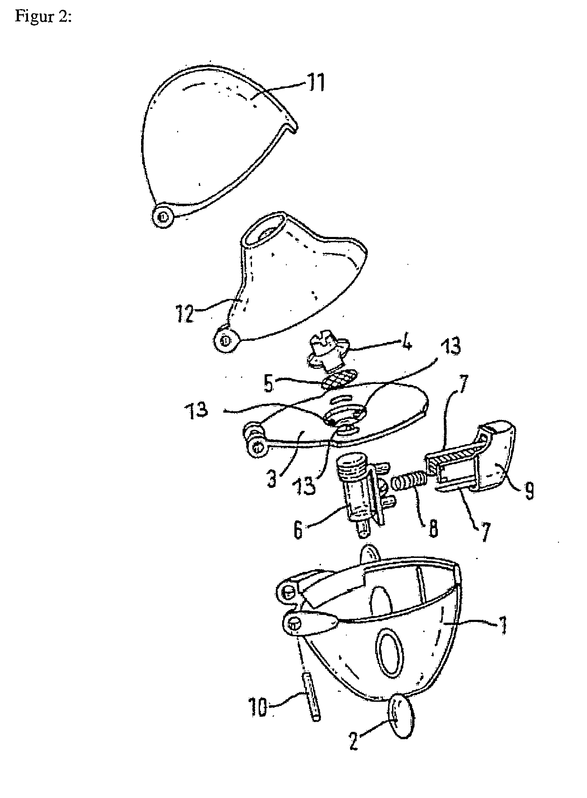

Besonders bevorzugt werden die das erfindungsgemäße Inhalationspulver enthaltenden Kapseln mit einem Inhalator appliziert, wie er in Figur 2 dargestellt ist.
Dieser Inhalator ist gekennzeichnet durch ein Gehäuse 1, enthaltend zwei Fenster 2, ein Deck 3, in dem sich Lufteinlassöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 9 vorgesehen ist, ein über eine Achse 10 klappbar mit dem Gehäuse 1, dem Deck 3 und einer Kappe 11 verbundenes Mundstück 12, sowie Luftdurchlasslöcher 13 zur Einstellung des Strömungswiderstandes.

Die vorliegende Erfindung betrifft ferner die Verwendung der das erfindungsgemäße kristalline Tiotropiumbromid-anhydrat enthaltenden Inhalationspulver zur Herstellung eines Arzneimittels zur Behandlung von Atemwegserkrankungen, insbesondere zur Behandlung von COPD und/oder Asthma, dadurch gekennzeichnet, dass der vorstehend beschriebene, in Figur 2 dargestellte Inhalator zur Anwendung gelangt.

Für die Anwendung der das erfindungsgemäße kristalline Tiotropiumbromid-anhydrat enthaltenden Inhalationspulver mittels pulverhaltiger Kapseln werden besonders bevorzugt solche Kapseln verwendet, deren Material ausgewählt ist aus der Gruppe der synthetischen Kunststoffe, besonders bevorzugt ausgewählt aus der Gruppe bestehend aus Polyethylen, Polycarbonat, Polyester, Polypropylen und Polyethylenterephthalat. Besonders bevorzugt sind als synthetische Kunststoffmaterialien Polyethylen, Polycarbonat oder Polyethylenterephthalat. Wird Polyethylen als eines der erfindungsgemäß besonders bevorzugten Kapselmaterialien verwendet, gelangt vorzugsweise Polyethylen mit einer Dichte zwischen 900 und 1000 kg/m³, bevorzugt von 940 - 980 kg/m³, besonders bevorzugt von etwa 960 - 970 kg/m³ (high-density Polyethylen) zur Anwendung.
Die synthetischen Kunststoffe im Sinne der Erfindung können vielseitig mittels dem im Stand der Technik bekannten Herstellverfahren verarbeitet werden. Bevorzugt im Sinne der Erfindung wird die spritzgusstechnische Verarbeitung der Kunststoffe. Besonders bevorzugt wird die Spritzgusstechnik unter Verzicht auf die Verwendung von Formtrennmitteln. Dieses Herstellverfahren ist wohldefiniert und durch eine besonders gute Reproduzierbarkeit gekennzeichnet.

Ein weiterer Aspekt der vorliegenden Erfindung betrifft vorstehend genannte Kapseln, die vorstehend genanntes Inhalationspulver enthalten. Diese Kapseln können etwa 1 bis 20 mg, bevorzugt etwa 3 bis 15 mg, besonders bevorzugt etwa 4 bis 12 mg Inhalationspulver enthalten. Erfindungsgemäß bevorzugte Formulierungen enthalten 4 bis 6 mg Inhalationspulver. Von erfindungsgemäß gleichrangiger Bedeutung sind Inhaltionskapseln, die die erfindungsgemäßen Formulierungen in einer Menge von 8 bis 12 mg enthalten.

Ferner betrifft die vorliegende Erfindung ein Inhalationskit, bestehend aus einer oder mehrerer der vorstehend beschriebenen, durch einen Gehalt an erfindungsgemäßem Inhalationspulver gekennzeichneten Kapseln in Verbindung mit dem Inhalator gemäß Figur 2.

Die vorliegende Erfindung betrifft ferner die Verwendung der vorstehend genannten, durch einen Gehalt an erfindungsgemäßem Inhalationspulver gekennzeichneten Kapseln, zur Herstellung eines Arzneimittels zur Behandlung von Atemwegserkrankungen, insbesondere zur Behandlung von COPD und/oder Asthma.

Die Darstellung von befüllten Kapseln, die die erfindungsgemäßen Inhalationspulver enthalten, erfolgt nach im Stand der Technik bekannten Verfahren durch Befüllung der leeren Kapseln mit den erfindungsgemäßen Inhalationspulvern.

### B.1.1. Beispiele für erfindungsgemäße Inhalationspulver

Die folgenden Beispiele dienen einer weitergehenden Erläuterung der vorliegenden Erfindung, ohne den Umfang der Erfindung allerdings auf die nachfolgenden beispielhaften Ausführungsformen zu beschränken.

### B.1.1.1. Ausgangsmaterialien

### Wirkstoff

Für die Herstellung der erfindungsgemäßen Inhalationspulver wird das erfindungsgemäße kristalline Tiotropiumbromid-anhydrat verwendet. Die Mikronisierung dieses Wirkstoffs erfolgt in Analogie zu im Stand der Technik bekannten Verfahren (siehe beispielsweise WO 03/078429 A1). Sofern im Rahmen der vorliegenden Erfindung auf die mittlere Teilchengröße des erfindungsgemäßen kristallinen Tiotropiumbromid-anhydrats Bezug genommen wird, erfolgt die Bestimmung derselben nach im Stand der Technik bekannten Meßmethoden (siehe beispielsweise WO 03/078429 A1, Abschnitt D.2).

### Hilfsstoff:

In den nachfolgenden Beispielen wird als Hilfsstoff Lactosemonohydrat verwendet. Dieser kann beispielsweise von der Firma Borculo Domo Ingredients, Borculo/NL unter der Produktbezeichnung *Lactochem Extra Fine Powder* bezogen werden. Die erfindungsgemäßen Spezifikationen für die Teilchengroße werden von dieser Lactosequalität erfüllt. Beispielsweise wurden in den nachfolgenden Beispielen Lactosechargen verwendet, die die folgenden Spezifikationen aufwiesen:

### B.1.1.2. Darstellung der erfindungsgemäßen Pulverformulierungen:

### I) Apparatives

Zur Herstellung der Inhalationspulver können beispielsweise die folgenden Maschinen und Geräte Verwendung finden:
Mischbehälter bzw. Pulvermischer: Turbulamischer 2 L, Typ 2C; Hersteller Willy A. Bachofen AG, CH-4500 Basel
Handsieb: 0,135 mm Maschenweite

Die Befüllung der leeren Inhaltionskapseln mittels Tiotropium-haltigen Inhaltionspulver kann händisch oder maschinell erfolgen. Es können nachstehende Geräte verwendet werden.

### Kapselfüllmaschine:

MG2, Typ G100, Hersteller: MG2 S.r.l, I-40065 Pian di Macina di Pianoro (BO), Italien

### Formulierungsbeispiel 1:

### Pulvermischung :

Zur Herstellung der Pulvermischung werden 299,39 g Hilfsstoff und 0,61 g mikronisiertes, kristallines Tiotropiumbromid-anhydrat eingesetzt.

Über ein Handsieb mit einer Maschenweite von 0,315 mm werden in einen geeigneten Mischbehälter ca. 40-45 g Hilfsstoff vorgelegt. Anschließend werden abwechselnd kristallines Tiotropiumbromid-anhydrat in Portionen von ca. 90-110 mg und Hilfsstoff in Portionen von etwa 40-45 g schichtweise eingesiebt. Die Zugabe des Hilfsstoffs und des Wirkstoffs erfolgt in 7 bzw. 6 Schichten.

Die eingesiebten Bestandteile werden anschließend gemischt (Mischen: 900 Umdrehungen). Die Endmischung wird noch zweimal über ein Handsieb gegeben und anschließend jeweils gemischt (Mischen: 900 Umdrehungen).

Gemäß der in Beispiel 1 beschriebenen Vorgehensweise können solche Inhalationspulver erhalten werden, die nach Befüllung der entsprechenden Kunststoffkapseln beispielsweise zu den nachstehenden Inhalationskapseln führen:

| **Formulierungsbeispiel 2:** | |
|---|---|
| Tiotropiumbromid-anhydrat: | 0,0113 mg |
| Lactose Monohydrat: | 5,4887 mg |
| Kapsel: | 100,0 mg |
| Total: | 105,5 mg |

| **Formulierungsbeispiel 3:** | |
|---|---|
| Tiotropiumbromid-anhydrat: | 0,0225 mg |
| Lactose Monohydrat: | 5,4775 mg |
| Polyethylen-Kapseln: | 100,0 mg |
| Total: | 105,5 mg |

| **Formulierungsbeispiel 4:** | |
|---|---|
| Tiotropiumbromid-anhydrat: | 0,0056 mg |
| Lactose Monohydrat: | 5,4944 mg |
| Polyethylen-Kapseln: | 100,0 mg |
| Total: | 105,5 mg |

| **Formulierungsbeispiel 5:** | |
|---|---|
| Tiotropiumbromid-anhydrat: | 0,0113 mg |
| Lactose Monohydrat:* | 5,4887 mg |
| Kapsel: | 100,0 mg |
| Total: | 105,5 mg |

| | |
|---|---|
| *) die Lactose enthält 5% spezifisch zugesetzten Feinanteil an mikronisiertem Lactosemonohydrat mit einer mittleren Teilchengröße von etwa 4µm. | |

| **Formulierungsbeispiel 6:** | |
|---|---|
| Tiotropiumbromid-anhydrat: | 0,0225 mg |
| Lactose Monohydrat:* | 5,4775 mg |
| Polyethylen-Kapseln: | 100,0 mg |
| Total: | 105,5 mg |

| | |
|---|---|
| *) die Lactose enthält 5% spezifisch zugesetzten Feinanteil an mikronisiertem Lactosemonohydrat mit einer mittleren Teilchengröße von etwa 4µm. | |

| **Formulierungsbeispiel 7:** | |
|---|---|
| Tiotropiumbromid-anhydrat: | 0,0056 mg |
| Lactose Monohydrat:* | 5,4944 mg |
| Polyethylen-Kapseln: | 100,0 mg |
| Total: | 105,5 mg |

| | |
|---|---|
| *) die Lactose enthält 5% spezifisch zugesetzten Feinanteil an mikronisiertem Lactosemonohydrat mit einer mittleren Teilchengröße von etwa 4µm. | |

### B.2. Treibgashaltige Aerosolsuspensionsaerosole enthaltend kristallines Tiotropiumbromid-anydrat

Das erfindungsgemäße kristalline Tiotropiumbromid-anhydrat kann gegebenenfalls auch in Form von treibgashaltigen Inhalationsaerosolen appliziert werden. Hierzu kommen insbesondere Aerosolsuspensionsformulierungen in Betracht.

Die vorliegende Erfindung betrifft daher ferner Suspensionen des erfindungsgemäßen kristallinen Tiotropiumbromid-anhydrats in den Treibgasen HFA 227 und/oder HFA 134a, gegebenenfalls in Mischung mit einem oder mehreren weiteren Treibgasen, vorzugsweise ausgewählt aus der Gruppe bestehend aus Propan, Butan, Pentan, Dimethylether, CHClF₂, CH₂F₂, CF₃CH₃, Isobutan, Isopentan und Neopentan.

Erfindungsgemäß bevorzugt sind solche Suspensionen, die als Treibgas nur HFA 227, ein Gemisch aus HFA 227 und HFA 134a oder nur HFA 134a enthalten.
Wird in den erfindungsgemäßen Suspensionsformulierungen ein Gemisch der Treibgase HFA 227 und HFA 134a eingesetzt, so sind die Gewichtsverhältnisse, in denen diese beiden Treibgaskomponenten zum Einsatz gelangen können, frei variabel.
Werden in den erfindungsgemäßen Suspensionsformulierungen neben den Treibgasen HFA 227 und/oder HFA 134a ein oder mehrere weitere Treibgase, ausgewählt aus der Gruppe bestehend aus Propan, Butan, Pentan, Dimethylether, CHClF₂, CH₂F₂, CF₃CH₃, Isobutan, Isopentan und Neopentan eingesetzt, so liegt der Anteil dieser weiteren Treibgaskomponente vorzugsweise unter 50 %, bevorzugt unter 40% besonders bevorzugt unter 30%.

Die erfindungsgemäßen Suspensionen enthalten vorzugsweise eine solche Menge an Tiotropiumbromid-anhydrat, dass der Anteil an Tiotropiumkation zwischen 0,001 bis 0,8%, erfindungsgemäß bevorzugt zeischen 0,08 bis 0,5%, besonders bevorzugt zwischen 0,2 bis 0,4% beträgt.
Bei den im Rahmen der vorliegenden Erfindung genannten prozentualen Angaben, handelt es sich sofern nicht gegenteilig angegeben stets um Gewichtsprozent.

Gegebenenfalls wird im Rahmen der vorliegenden Erfindung statt des Begriffs Suspension auch der Begriff Suspensionsformulierung verwendet. Beide Begriffe sind im Rahmen der vorliegenden Erfindung als gleichbedeutend anzusehen.

Die erfindungsgemäßem treibgashaltigen Inhalationsaerosole bzw. Suspensionsformulierungen können ferner weitere Bestandteile wie oberflächenaktive Mittel (Tenside, Surfactants), Adjuvantien, Antioxidantien oder Geschmacksmittel enthalten.

Die in den erfindungsgemäßen Suspensionen gegebenenfalls enthaltenen oberflächenaktiven Mittel (Tenside, Surfactants) sind vorzugsweise ausgewählt aus der Gruppe bestehend aus Polysorbat 20, Polysorbat 80, Myvacet 9-45, Myvacet 9-08, Isopropylmyristat, Ölsäure, Propylenglycol, Polyethylenglycol, Brij, Ethyloleat, Glyceryltrioleat Glycerylmonolaurat, Glycerylmonooleat, Glycerylmonosterat, Glycerylmonoricinoleate, Cetylalcohol, Sterylalkohol, Cetylpyridinumchlorid, Blockpolymere, natürliches Öl, Ethanol und Isopropanol. Von den vorstehend genannten Suspensionshilfsstoffen gelangen bevorzugt zur Anwendung Polysorbat 20, Polysorbat 80, Myvacet 9-45, Myvacet 9-08 oder Isopropylmyristat. Besonders bevorzugt gelangen zur Anwendung Myvacet 9-45 oder Isopropylmyristat.

Sofern in den erfindungsgemäßen Suspensionen oberflächenaktive Mittel enthalten sind, werden diese vorzugsweise in einem Anteil von 0,0005 - 1 %, besonders bevorzugt 0,005 - 0,5 % eingesetzt.

Die in den erfindungsgemäßen Suspensionen gegebenenfalls enthaltenen Adjuvantien sind vorzugsweise ausgewählt aus der Gruppe bestehend aus Alanin, Albumin, Ascorbinsäure, Aspartam, Betain, Cystein, Phosphorsäure, Salpetersäure, Salzsäure, Schwefelsäure und Zitronensäure. Besonders bevorzugt gelangen dabei zur Anwendung Ascorbinsäure, Phosphorsäure, Salzsäure oder Zitronensäure, besonders bevorzugt Salzsäure oder Zitronensäure.

Sofern in den erfindungsgemäßen Suspensionen Adjuvantien enthalten sind, werden diese vorzugsweise in einem Anteil von 0,0001-1,0 %, bevorzugt 0,0005-0,1 %, besonders bevorzugt 0,001-0,01 % eingesetzt, wobei ein Anteil von 0,001-0,005 % erfindungsgemäß von besonderer Bedeutung ist.

Die in den erfindungsgemäßen Suspensionen gegebenenfalls enthaltenen Antioxidantien sind vorzugsweise ausgewählt aus der Gruppe bestehend aus Ascorbinsäure, Zitronensäure, Natriumedetat, Editinsäure Tocopherolen, Butylhydroxytoluol, Butylhydroxyanisol und Ascorbylpalmitat, wobei Tocopherole, Butylhydroxytoluol, Butylhydroxyanisol oder Ascorbylpalmitat bevorzugt zum Einsatz gelangen.

Die in den erfindungsgemäßen Suspensionen gegebenenfalls enthaltenen Geschmacksmittel sind vorzugsweise ausgewählt aus der Gruppe bestehend aus Pefferminz, Sacharin, Dentomint, Aspartam und etherischen Ölen (beispielsweise Zimt, Anis, Menthol, Campher), wobei beispielsweise Pefferminz oder Dentomint® besonders bevorzugt sind.

Im Hinblick auf die inhalative Applikation ist es erforderlich, die Wirkstoffe in feinteiliger Form bereitzustellen. Das erfindungsgemäße kristalline Tiotropiumbromid-anhydrat wird dazu nach im Stand der Technik bekannten Verfahren in feinteiliger Form gewonnen.

Verfahren zur Mikronisierung von Wirkstoffen sind im Stand der Technik bekannt. Vorzugsweise weist der Wirkstoff nach Mikronisierung eine mittlere Teilchengröße von 0,5 bis 10µm, bevorzugt von 1 bis 6µm, besonders bevorzugt von 1,5 bis 5µm auf. Vorzugsweise weisen wenigstens 50%, bevorzugt wenigstens 60%, besonders bevorzugt wenigstens 70% der Wirkstoffteilchen eine Teilchengröße auf, die innerhalb der vorstehend genannten Größenbereiche liegt. Besonders bevorzugt liegen wenigstens 80%, höchst bevorzugt wenigstens 90% der Wirkstoffteilchen mit ihrer Partikelgröße in den vorstehend genannten Bereichen.

Ein weiterer Aspekt der vorliegenden Erfindung zielt auf Suspensionen, die lediglich einer der beiden erfindungsgemäßen Wirkstoffe ohne weitere Zusätze enthalten.

Zur Herstellung der erfindungsgemäßen Suspensionen kann nach im Stand der Technik bekannten Verfahren vorgegangen werden. Hierzu werden die Bestandteile der Formulierung mit dem oder den Treibgasen (ggf. bei niedrigen Temperaturen) gemischt und in geeignete Behälter abgefüllt.

Die vorstehend genannten erfindungsgemäßen treibgashaltigen Suspensionen können mittels im Stand der Technik bekannten Inhalatoren (pMDIs = pressurized metered dose inhalers) appliziert werden. Dementsprechend betrifft ein weiterer Aspekt der vorliegenden Erfindung Arzneimittel in Form von wie vorstehend beschriebenen Suspensionen in Verbindung mit einem oder mehreren zur Verabreichung dieser Suspensionen geeigneten Inhalatoren. Ferner betrifft die vorliegende Erfindung Inhalatoren, dadurch gekennzeichnet, daß sie vorstehend beschriebene erfindungsgemäße treibgashaltige Suspensionen enthalten.

Die vorliegende Erfindung betrifft ferner Behälter (Kartuschen), die ausgestattet mit einem geeigneten Ventil in einem geeigneten Inhalator zur Anwendung gelangen können und die eine der vorstehend genannten erfindungsgemäßen treibgashaltigen Suspensionen enthalten. Geeignete Behälter (Kartuschen) und Verfahren zur Abfüllung dieser Kartuschen mit den erfindungsgemäßen treibgashaltigen Suspensionen sind aus dem Stand der Technik bekannt.

Aufgrund der pharmazeutischen Wirksamkeit von Tiotropium betrifft die vorliegende Erfindung ferner die Verwendung der erfindungsgemäßen Suspensionen zur Herstellung eines inhalativ oder nasal applizierbaren Arzneimittels, bevorzugt zur Herstellung eines Arzneimittels zur inhalativen oder nasalen Behandlung von Erkrankungen, in denen Anticholinergika einen therapeutischen Nutzen entfalten können.

Besonders bevorzugt betrifft die vorliegende Erfindung ferner die Verwendung der erfindungsgemäßen Suspensionen zur Herstellung eines Arzneimittels zur inhalativen Behandlung von Atemwegserkrankungen, bevorzugt von Asthma oder COPD.

Die nachfolgenden Beispiele dienen der exemplarischen, weitergehenden Illustration der vorliegenden Erfindung, ohne selbige auf deren Inhalt zu beschränken.

### B.2.1 Beispiele für Aerosolsuspensionsformulierungen

Suspensionen enthaltend neben Wirkstoff und Treibgas weitere Bestandteile:

| **Formulierungsbeispiel 8:** | |
|---|---|
| **Bestandteile** | **Konzentration [% w/w]** |
| Tiotropiumbromid-anhydrat | 0,04 |
| Ölsäure | 0,005 |
| HFA-227 | 99,955 |

| **Formulierungsbeispiel 9:** | |
|---|---|
| **Bestandteile** | **Konzentration [% w/w]** |
| Tiotropiumbromid-anhydrat | 0,02 |
| Ölsäure | 0,01 |
| HFA-227 | 60,00 |
| HFA-134a | 39,97 |

| **Formulierungsbeispiel 10:** | |
|---|---|
| **Bestandteile** | **Konzentration [% w/w]** |
| Tiotropiumbromid-anhydrat | 0,02 |
| Isopropylmyristat | 1,00 |
| HFA-227 | 98,98 |

| **Formulierungsbeispiel 11:** | |
|---|---|
| **Bestandteile** | **Konzentration [% w/w]** |
| Tiotropiumbromid-anhydrat | 0,02 |
| Myvacet 9-45 | 0,3 |
| HFA-227 | 99,68 |

| **Formulierungsbeispiel 12:** | |
|---|---|
| **Bestandteile** | **Konzentration [% w/w]** |
| Tiotropiumbromid-anhydrat | 0,02 |
| Myvacet 9-45 | 0,1 |
| HFA-227 | 60,00 |
| HFA-134a | 39,88 |

| **Formulierungsbeispiel 13:** | |
|---|---|
| **Bestandteile** | **Konzentration [% w/w]** |
| Tiotropiumbromid-anhydrat | 0,04 |
| Polysorbat 80 | 0,04 |
| HFA-227 | 99,92 |

| **Formulierungsbeispiel 14:** | |
|---|---|
| **Bestandteile** | **Konzentration [% w/w]** |
| Tiotropiumbromid-anhydrat | 0,01 |
| Polysorbat 20 | 0,20 |
| HFA-227 | 99,78 |

| **Formulierungsbeispiel 15:** | |
|---|---|
| **Bestandteile** | **Konzentration [% w/w]** |
| Tiotropiumbromid-anhydrat | 0,04 |
| Myvacet 9-08 | 01,00 |
| HFA-227 | 98,96 |

| **Formulierungsbeispiel 16:** | |
|---|---|
| **Bestandteile** | **Konzentration [% w/w]** |
| Tiotropiumbromid-anhydrat | 0,02 |
| Isopropylmyristat | 0,30 |
| HFA-227 | 20,00 |
| HFA-134a | 79,68 |

Suspensionen enthaltend lediglich Wirkstoff und Treibgas:

| **Formulierungsbeispiel 17:** | |
|---|---|
| **Bestandteile** | **Konzentration [% w/w]** |
| Tiotropiumbromid-anhydrat | 0,02 |
| HFA-227 | 60,00 |
| HFA-134a | 39,98 |

| **Formulierungsbeispiel 18:** | |
|---|---|
| **Bestandteile** | **Konzentration [% w/w]** |
| Tiotropiumbromid-anhydrat | 0,02 |
| HFA-227 | 99,98 |

| **Formulierungsbeispiel 19:** | |
|---|---|
| **Bestandteile** | **Konzentration [% w/w]** |
| Tiotropiumbromid-anhydrat | 0,02 |
| HFA-134a | 99,98 |

| **Formulierungsbeispiel 20:** | |
|---|---|
| **Bestandteile** | **Konzentration [% w/w]** |
| Tiotropiumbromid-anhydrat | 0,02 |
| HFA-227 | 99,98 |

| **Formulierungsbeispiel 21:** | |
|---|---|
| **Bestandteile** | **Konzentration [% w/w]** |
| Tiotropiumbromid-anhydrat | 0,02 |
| HFA-134a | 99,98 |

| **Formulierungsbeispiel 22:** | |
|---|---|
| **Bestandteile** | **Konzentration [% w/w]** |
| Tiotropiumbromid-anhydrat | 0,02 |
| HFA-227 | 20,00 |
| HFA-134a | 79,98 |

| **Formulierungsbeispiel 23:** | |
|---|---|
| **Bestandteile** | **Konzentration [% w/w]** |
| Tiotropiumbromid-anhydrat | 0,04 |
| HFA-227 | 40,00 |
| HFA-134a | 59,96 |

| **Formulierungsbeispiel 24:** | |
|---|---|
| **Bestandteile** | **Konzentration [% w/w]** |
| Tiotropiumbromid-anhydrat | 0,04 |
| HFA-227 | 80,00 |
| HFA-134a | 19,96 |

## Patentansprüche

1. Wasserfreies, kristallines Tiotropiumbromid, welches **dadurch gekennzeichnet ist, dass** es im Röntgenpulverdiagramm unter anderen die charakteristischen Werte d= 6,02 Ä; 4,95 Å; 4,78 Å; ; 3,93 Ä und 3,83 Å aufweist.

2. Arzneimittel **gekennzeichnet durch** einen Gehalt an kristallinem, wasserfreiem Tiotropiumbromid nach Anspruch 1.

3. Arzneimittel nach Anspruch 2, **dadurch gekennzeichnet, daß** es sich um ein Inhalationspulver handelt.

4. Arzneimittel nach Anspruch 3, **dadurch gekennzeichnet, daß** es ein Inhalationspulver ist, welches neben dem wasserfreien, kristallinen Tiotropiumbromid einen oder mehrere geeignete physiologisch unbedenkliche Hilfsstoffe ausgewählt aus der Gruppe der Monosaccharide, der Disaccharide, der Oligo- und Polysaccharide, der Polyalkohole, der Cyclodextrine, der Aminosäuren oder auch der Salze oder Mischungen dieser Hilfsstoffe miteinander enthält.

5. Arzneimittel nach Anspruch 4, **dadurch gekennzeichnet, dass** der Hilfsstoff ausgewählt ist aus der Gruppe bestehend aus Glucose, Fructose, Arabinose, Lactose, Saccharose, Maltose, Trehalose, Dextrane, Dextrine, Maltodextrin, Stärke, Cellulose, Sorbit, Mannit, Xylit, α-Cydodextrin, β-Cyclodextrin, χ-Cyclodextrin, Methyl-β-Cyclodextrin, Hydroxypropyl-β-Cyclodextrin, Argininhydrochlorid, Natriumchlorid oder Calciumcarbonat, oder Mischungen davon.

6. Arzneimittel nach Anspruch 4 oder 5, **dadurch gekennzeichnet, daß** es zwischen 0,01 bis 2 % Tiotropium enthält.

7. Kapseln **gekennzeichnet durch** einen Gehalt an Inhaltionspulver nach einem der Ansprüche 3 bis 6.

8. Arzneimittel nach Anspruch 2, **dadurch gekennzeichnet, daß** es sich um eine treibgashaltige Aerosolsuspensionsformulierung handelt.

9. Verwendung von kristallinem, wasserfreiem Tiotropiumbromid nach Anspruch 1 zur Herstellung eines Arzneimittels zur Behandlung von Atemwegserkrankungen.

10. Verwendung nach Anspruch 9, **dadurch gekennzeichnet, daß** es sich bei den Atemwegserkrankungen um Asthma oder COPD handelt.

## Claims

1. Anhydrous crystalline tiotropium bromide which is **characterised in that** in the X-ray powder diagram it has the characteristic values d= 6.02 Å; 4.95 Å; 4.78 Å; 3.93 Å and 3.83 Å, *inter alia.*

2. Medicament, **characterised in that** it contains crystalline anhydrous tiotropium bromide according to claim 1.

3. Medicament according to claim 2, **characterised in that** it is an inhalable powder.

4. Medicament according to claim 3, **characterised in that** it is an inhalable powder which contains in addition to the anhydrous crystalline tiotropium bromide one or more suitable physiologically acceptable excipients selected from among the monosaccharides, the disaccharides, the oligo- and polysaccharides, the polyalcohols, the cyclodextrins, the amino acids or the salts or mixtures of these excipients with one another.

5. Medicament according to claim 4, **characterised in that** the excipient is selected from the group consisting of glucose, fructose, arabinose, lactose, saccharose, maltose, trehalose, dextrans, dextrins, maltodextrin, starch, cellulose, sorbitol, mannitol, xylitol, α-cyctodextrin, β-cyclodextrin, χ-cyclodextrin, methyl-β-cyclodextrin, hydroxypropyl-β-cyclodextrin, arginine hydrochloride, sodium chloride or calcium carbonate, or mixtures thereof.

6. Medicament according to claim 4 or 5, **characterised in that** it contains between 0.01 and 2 % tiotropium.

7. Capsules, **characterised in that in that** they contain an inhalable powder according to one of claims 3 to 6.

8. Medicament according to claim 2, **characterised in that** it is a propellant-containing aerosol suspension formulation.

9. Use of crystalline anhydrous tiotropium bromide according to claim 1 for preparing a pharmaceutical composition for the treatment of respiratory complaints.

10. Use according to claim 9, **characterised in that** the respiratory complaints are asthma or COPD.

## Revendications

1. Bromure de tiotropium cristallin anhydre qui est **caractérisé en ce qu'**il présente dans le diagramme de diffraction des rayons X de poudre entre autres les valeurs caractéristiques d = 6,02 Å ; 4,95 Å ; 4,78 Å ; 3,93 Å et 3,83 Å.

2. Médicament **caractérisé par** une teneur en bromure de tiotropium anhydre cristallin selon la revendication 1.

3. Médicament selon la revendication 2 **caractérisé en ce qu'**il s'agit d'une poudre pour inhalation.

4. Médicament selon la revendication 3 **caractérisé en ce que** c'est une poudre pour inhalation qui contient, outre le bromure de tiotropium cristallin anhydre, un ou plusieurs adjuvants physiologiquement acceptables appropriés choisis dans le groupe des monosaccharides, des disaccharides, des oligo- et polysaccharides, des polyalcools, des cyclodextrines, des aminoacides ou encore des sels ou des mélanges de ces adjuvants entre eux.

5. Médicament selon la revendication 4 **caractérisé en ce que** l'adjuvant est choisi dans le groupe consistant en le glucose, le fructose, l'arabinose, le lactose, le saccharose, le maltose, le tréhalose, les dextranes, les dextrines, la maltodextrine, l'amidon, la cellulose, le sorbitol, le mannitol, le xylitol, l'α-cyclodextrine, la β-cyclodextrine, la χ-cyclodextrine, la méthyl-β-cyclodextrine, l'hydroxypropyl-β-cyclodextrine, le chlorhydrate d'arginine, le chlorure de sodium et le carbonate de calcium, ou leurs mélanges.

6. Médicament selon la revendication 4 ou 5 **caractérisé en ce qu'**il contient 0,01 à 2 % de tiotropium.

7. Capsules **caractérisées par** une teneur en poudre pour inhalation selon l'une des revendications 3 à 6.

8. Médicament selon la revendication 2 **caractérisé en ce qu'**il s'agit d'une formulation de suspension d'aérosol contenant un gaz propulseur.

9. Utilisation du bromure de tiotropium anhydre cristallin selon la revendication 1 pour la production d'un médicament pour le traitement des maladies des voies respiratoires.

10. Utilisation selon la revendication 9 **caractérisée en ce que** les maladies des voies respiratoires sont l'asthme ou la COPD.
